# EUROPEAN PATENT APPLICATION

(11) **EP 1 300 462 A1**
(43) Date of publication of application: **09.04.2003**
(21) Application number: 01945682.1
(22) Date of filing: 28.06.2001
(51) Int. Cl.: C12M 1/20, C12M 1/38, C12Q 1/68, B01J 19/00

(54) **REACTION VESSEL, REACTION DEVICE AND TEMPERATURE CONTROL METHOD FOR REACTION LIQUID**

(30) Priority: 30.06.2000 JP 2000197821
(71) Applicant: PRECISION SYSTEM SCIENCE CO., LTD., Matsudo-shi, Chiba, 271-0064 (JP)
(72) Inventor: TAJIMA, Hideji, Matsudo-shi, Chiba 271-0064 (JP)
(74) Representative: Bohnenberger, Johannes, Dr.
(86) International application number: JP0105598
(87) International publication number: WO02002736

(57) **Abstract**

It is an object of the present invention to provide a reaction vessel which makes it possible to control the temperature of the reaction solution accommodated in the reaction chamber with a quick response, without any need for centrifuging when the reaction solution is accommodated in the reaction chamber, and which also makes it possible to cause the reaction to proceed even when the amount of reaction solution accommodated in the reaction chamber is extremely small. The present invention provides a reaction vessel comprising a reaction vessel main body which has a reaction chamber that has an opening part in the upper end and that can accommodate a reaction solution, and a cover member which can seal the opening part of the abovementioned reaction chamber, wherein the abovementioned cover member has a pressing part that can press the reaction solution accommodated in the abovementioned reaction chamber.

## Description

### TECHNICAL FIELD

The present invention relates to a reaction vessel (especially a reaction vessel which can be suitable used for a reaction requiring temperature control), a reaction apparatus utilizing this reaction vessel, and a reaction solution temperature control method.

### BACKGROUND ART

A polymerase chain reaction (hereafter referred to as "PCR") is a technique which amplifies target nucleic acids by a rise and fall in temperature utilizing a heat-resistant polymerase and primers. This technique is widely used in fields such as genetic engineering, biological test methods and detection methods and the like.

The principle of PCR lies in the fact that target DNA is amplified in a geometrical progression by numerous repetitions of a cycle according to a thermal profile (rise and fall of temperature) that is set in three stages, i. e., a first stage in which the temperature is maintained at a temperature that dissociates double-stranded DNA containing a target DNA sequence into a single strand, a second stage in which the temperature is maintained at a temperature that causes annealing of forward and reverse primers with the dissociated single-stranded DNA, and a third stage in which the temperature is maintained at a temperature at which a complementary DNA chain is synthesized with the single-stranded DNA by the DNA polymerase.

For example, a PCR can be caused to proceed by reacting a reaction solution containing double-stranded DNA that includes a target DNA sequence, an excess of a pair of primers and a heat-resistant polymerase for 30 to 40 cycles with one cycle comprising 30 seconds at 95°C, 30 seconds at 65°C and 1 minute at 72°C. At 95°C, the double-stranded DNA dissociates to become single-stranded DNA. Next, when the reaction solution is cooled to an appropriate temperature in accordance with the base sequences of the primers (65°C in the above example), the primers and the single-stranded DNA are annealed. Next, when the temperature is raised to the reaction temperature of the polymerase (72°C in the above example), a DNA synthesis reaction is caused to proceed by the polymerase.

Thus, in a PCR, control of the temperature of the reaction solution is important. Accordingly, such a PCR is ordinarily performed using a thermostat apparatus that allows programming of the temperature control, and a reaction vessel that can be used in such an apparatus.

Most commonly, an apparatus is used in which micro-tubes are mounted tightly in holes of a metal block equipped with a heating/cooling apparatus, and a cycle of heating (dissociation of the double-stranded DNA), cooling (annealing of the primers) and heating (chain extension reaction by the polymerase) is repeated for the reaction solution in the micro-tubes via the metal block. Cooling systems for the metal block include cooling systems of two types, i. e., systems using a compressor, and Peltier cooling systems. Recently, apparatuses have also been available in which the micro-tubes are moved together with a rack rather than using a metal block, and in which the micro-tubes are successively immersed in three liquid-phase or solid-phase incubators with independent temperatures, so that a cycle consisting of heating (dissociation of the double-stranded DNA), cooling (annealing of the primers) and heating (chain extension reaction by the polymerase) is repeated.

In order to allow the treatment of numerous specimens at one time in cases where the number of specimens is large, as when a PCR is performed for the purpose of screening, apparatuses in which PCRs for 96 specimens can be performed at one time using a PCR micro-titer plate (96 wells) have also been developed.

In particular, there has recently been an increased need for the efficient treatment of numerous specimens in parallel by automating a series of operations comprising the preparation of samples containing target nucleic acids (extraction of nucleic acids from cells), amplification of these target nucleic acids by PCR, and analysis of these target nucleic acids, in order to treat numerous specimens with good efficiency in genetic diagnosis and the genome project. Furthermore, in order to automate this series of operations and treat numerous specimens efficiently in parallel, it is necessary first of all to minimize the time required for the PCR, and secondly to minimize the quantity of each specimen required for the PCR.

However, in the case of conventional PCR reaction apparatuses and PCR reaction vessels, since the object is to perform a PCR by means of typical temperature control in which a reaction is performed for 30 to 40 cycles with one cycle comprising 30 seconds at 95°C, 30 seconds at 65°C and 1 minute at 72°C, it is difficult to achieve the object of minimizing the time required for the PCR using such a conventional PCR reaction apparatus and PCR reaction vessel. For example, in cases where a reaction is performed for 30 to 40 cycles using a conventional PCR reaction apparatus and PCR reaction vessel with one cycle comprising 30 seconds at 95°C, 30 seconds at 65°C and 1 minute at 72°C, a time of approximately 1 hour is required in order to complete the PCR.

Furthermore, in the case of a conventional PCR reaction apparatus and PCR reaction vessel, if the amount of specimen (reaction solution) is too small, there may be cases in which the solvent (ordinarily water) in the reaction solution evaporates during the PCR so that the reaction stops. The reasons for this are as follows: since the contact area between air and the reaction solution in the reaction chamber (e. g., micro-tube or micro-titer plate well) in which the PCR proceeds is large, the solvent in the reaction solution is in an environment in which evaporation tends to occur; furthermore, since the temperature of the inside walls of the reaction chamber is non-uniform, so that there are portions of the inside walls of the reaction chamber where the temperature is lower than the temperature of the reaction solution (e. g., the upper part of a micro-tube or upper part of a micro-titer plate well), the evaporated solvent is liquefied in these areas. Generally, in order to prevent evaporation of the solvent in the reaction solution, a layer of mineral oil or the like is superimposed on the reaction solution; however, if the amount of the reaction solution is very small, it is difficult to sample the reaction solution beneath the mineral oil following the reaction. Accordingly, it is difficult to achieve the object of minimizing the amount of reaction solution using a conventional PCR reaction apparatus and PCR reaction vessel.

Under such conditions, an apparatus has been developed in which a small amount of reaction solution is enclosed inside a micro-capillary which has a large surface area and good thermal conductivity, and heating and cooling are performed by means of a hot air draft from a halogen lamp or the like as a heat source and a cooling air draft in room temperature. For example, LightCycler (manufactured by Roche Molecular Biochemicals) is marketed as an apparatus of this type. In the case of this apparatus, temperature control of approximately 20°C/sec can be achieved by utilizing micro-capillaries that have a large surface area and a good thermal conductivity; accordingly, a time of only about 30 to 60 seconds is required for one cycle, so that 30 cycles can be completed in approximately 15 to 30 minutes. Furthermore, since micro-capillaries are utilized, a PCR using a very small amount of reaction solution, i. e., approximately 5 to 20 µl, can be realized.

Thus, a PCR reaction apparatus using a micro-capillary as a PCR reaction vessel can shorten the time required for the PCR by accomplishing temperature control of the reaction solution with a quick response, and also makes it possible to reduce the amount of reaction solution required for the PCR to an extremely small amount. Accordingly, such a PCR reaction apparatus is extremely useful when a PCR is performed alone.

### DISCLOSURE OF THE INVENTION

In the case of a PCR reaction apparatus comprising a micro-capillary as a PCR reaction vessel, an operation in which the reaction solution is added to plastic containers disposed on the upper parts of glass capillaries, and sealed by means of plastic stoppers, after which a centrifuge is used to move the reaction solution into the glass capillaries from the plastic containers, and the respective capillaries are then removed from the centrifuge and placed in the reaction apparatus, is required when the reaction solution is enclosed inside the micro-capillaries. Furthermore, if air is admixed when the reaction solution is enclosed inside the micro-capillaries, this air will expand as a result of the heating that is performed in the process of the PCR, so that the reaction solution will move through the micro-capillaries, thus causing a drop in the amplification efficiency of the PCR. Consequently, it is necessary to pay close attention when the reaction solution is enclosed in the micro-capillaries.

Accordingly, it is difficult to utilize a PCR reaction apparatus comprising a micro-capillary as a PCR reaction vessel for the automation of the series of operations comprising the preparation of samples containing target nucleic acids (extraction of nucleic acids from cells), amplification of the target nucleic acids by means of a PCR, and analysis of the target nucleic acids.

Consequently, a first object of the present invention is to provide a reaction vessel which makes it possible to control the temperature of the reaction solution accommodated in the reaction chamber with a quick response, without any need for centrifuging when the reaction solution is accommodated in the reaction chamber, and which also makes it possible to cause the reaction to proceed even when the amount of reaction solution accommodated in the reaction chamber is extremely small.

Furthermore, a second object of the present invention is to provide a reaction apparatus comprising the abovementioned reaction vessel.

Furthermore, a third object of the present invention is to provide a reaction solution temperature control method which can control the temperature of the reaction solution accommodated in the reaction chamber with a quick response.

(1) In order to achieve the abovementioned first object, the present invention provides a reaction vessel comprising a reaction vessel main body which has a reaction chamber that has an opening part in the upper end and that can accommodate a reaction solution, and a cover member which can seal the opening part of the reaction chamber, wherein the cover member has a pressing part that can press the reaction solution accommodated in the reaction chamber.

In the reaction vessel of the present invention, the reaction chamber has an opening part in the upper end, and can accommodate a reaction solution, and the reaction solution is added from the opening part in the upper end of the reaction chamber and accommodated in the reaction chamber. The reaction chamber is the place where the desired reaction is caused to proceed, and reagents and the like that cause the desired reaction to proceed are contained in the reaction solution that is accommodated in the reaction chamber. In the reaction vessel of the present invention, the cover member is mounted on the reaction vessel main body after the reaction solution has been accommodated in the reaction chamber.

In the reaction vessel of the present invention, the reaction chamber may be any chamber that has an opening part in the upper end, and that can accommodate a reaction solution; there are no particular restrictions on the structure of the reaction chamber. In the reaction vessel of the present invention, there is no need for the reaction chamber to have a capillary-like structure, and there is no need for centrifuging when the reaction solution is accommodated in the reaction chamber. The reaction vessel of the present invention is devised so that when the cover member is mounted on the reaction vessel main body, the pressing part of the cover member advances into the interior of the reaction chamber from the opening part of the reaction chamber, and presses the reaction solution accommodated in the reaction chamber. Accordingly, it is desirable that the reaction chamber has a structure that allows easy entry of the pressing part of the cover member. Furthermore, it is desirable that the reaction chamber has a structure which is such that the reaction solution added from the opening part can reach the bottom surface of the reaction chamber without any force other than gravity being applied to the reaction solution in the downward direction. Accordingly, in the reaction vessel of the present invention, a reaction chamber which has a capillary-like structure is actually inappropriate.

In the reaction vessel of the present invention, the cover member is a member that can seal the opening part of the reaction chamber, and when the cover member is mounted on the reaction vessel main body, the opening part of the reaction chamber is sealed by the cover member. As a result, contamination of the reaction solution accommodated in the reaction chamber can be prevented, so that the desired reaction can be accurately performed in the reaction chamber. In cases where the reaction vessel main body comprises a plurality of reaction chambers, the opening parts of the respective reaction chambers are sealed by a cover member, so that the admixture of the reaction solution contained in one reaction chamber with the reaction solution contained in other reaction chambers can be prevented, thus allowing the desired reaction to be accurately performed in the respective reaction chambers.

In the reaction vessel of the present invention, the cover member has a pressing part that presses the reaction solution accommodated in the reaction chamber. When the cover member is mounted on the reaction vessel main body, the pressing part of the cover member advances into the interior of the reaction chamber from the opening part of the reaction chamber, contacts the reaction solution accommodated in the reaction chamber, and presses the reaction solution. The pressing part of the cover member is disposed so that this pressing part can press the reaction solution during the process of the mounting of the cover member on the reaction vessel main body and/or in the state in which the cover member has been mounted on the reaction vessel main body. Accordingly, during the process of the mounting of the cover member on the reaction vessel main body and/or in the state in which the cover member has been mounted on the reaction vessel main body, the reaction solution accommodated in the reaction chamber has a contact surface with the reaction chamber and a contact surface with the cover member. As a result, not only the movement of heat via the contact surface between the reaction solution and the reaction chamber, but also the movement of heat via the contact surface between the reaction solution and the cover member, is possible, so that the temperature of the reaction solution can be controlled more quickly than is possible before the reaction solution is pressed. For example, heat can be caused to move into the reaction solution from the reaction chamber and the cover member via the contact surface between the reaction solution and the reaction chamber and the contact surface between the reaction solution and the cover member by raising the temperature of the reaction chamber and the cover member, so that the temperature of the reaction solution can be raised. Furthermore, heat can be caused to move into the reaction chamber and cover member from the reaction solution via the contact surface between the reaction solution and the reaction chamber and the contact surface between the reaction solution and the cover member by lowering the temperature of the reaction chamber and the cover member, so that the temperature of the reaction solution can be lowered.

In the reaction vessel of the present invention, as long as the pressing part of the cover member is able to press the reaction solution during the process of the mounting of the cover member on the reaction vessel main body and/or in the state in which the cover member has been mounted on the reaction vessel main body, the pressing part of the cover member may be disposed so that the reaction solution is pressed in a state in which a fixed contact surface between the reaction solution and the pressing part of the cover member is maintained, or may be disposed so that the reaction solution is pressed while the contact surface between the reaction solution and the pressing part of the cover member is increased.

In the reaction vessel of the present invention, when the cover member is mounted on the reaction vessel main body, the pressing part of the cover member advances into the interior of the reaction chamber from the opening part of the reaction chamber, so that gases such as air and the like that are present inside the reaction chamber are pushed out of the reaction chamber, and the opening part of the reaction chamber is sealed in this state. Accordingly, the amount of gases such as air and the like present inside the reaction chamber is decreased compared to the state prior to the mounting of the cover member. Furthermore, since the pressing part of the cover member that has advanced into the interior of the reaction chamber contacts the reaction solution accommodated in the reaction chamber, the contact area between the reaction solution and gases such as air and the like that are present in the reaction chamber is reduced compared to the state prior to the mounting of the cover member. Thus, when the cover member is mounted on the reaction vessel main body, the amount of gases such as air and the like present inside the reaction chamber is reduced, and the contact area between the reaction solution and gases such as air and the like present inside the reaction chamber is also reduced; accordingly, when the desired reaction is caused to proceed inside the reaction chamber, the evaporation of the reaction solution into gases such as air and the like present inside the reaction chamber can be suppressed. As a result, the reaction can be caused to proceed even if the amount of reaction solution accommodated in the reaction chamber is an extremely small amount.

In the reaction vessel of the present invention, the desired reaction is caused to proceed inside the reaction chamber. When this desired reaction is caused to proceed inside the reaction chamber, the temperature of the reaction solution is controlled as necessary. The control of the temperature of the reaction solution is accomplished mainly by the movement of heat via the contact surface between the reaction solution and the reaction chamber and the contact surface between the reaction solution and the cover member. In cases where gases such as air or the like are present inside the reaction chamber, the movement of heat via these gases such as air or the like may also occur. The control of the temperature of the reaction solution is ordinarily performed after the cover member has been mounted on the reaction vessel main body. In cases where the control of the temperature of the reaction solution is performed after the cover member has been mounted on the reaction vessel main body, the temperature of the reaction solution can be controlled by the movement of heat via the contact surface between the reaction solution and the reaction chamber and the contact surface between the reaction solution and the cover member. Control of the temperature of the reaction solution may also be performed before the cover member is mounted on the reaction vessel main body and/or during the process of the mounting of the cover member on the reaction vessel main body. Before the cover member is mounted on the reaction vessel main body, the temperature of the reaction solution can be controlled by the movement of heat via the contact surface between the reaction solution and the reaction chamber. During the process of the mounting of the cover member on the reaction vessel main body, the temperature of the reaction solution can be controlled by the movement of heat via the contact surface between the reaction solution and the reaction chamber and (in a state where the pressing part of the cover member is pressing the reaction solution) via the contact surface between the reaction solution and the cover member.

In the reaction vessel of the present invention, there are no particular restrictions on the reaction that is caused to proceed inside the reaction chamber; however, the reaction vessel of the present invention can be suitable used for reactions (e. g., enzyme reactions) in which there is a need to control the temperature of the reaction solution at which the reaction is initiated, caused to proceed or stopped, and the reaction vessel of the present invention is especially suitable for use in reactions (e. g., PCR) in which there is a need to control the temperature of the reaction solution periodically or over time when the reaction is caused to proceed. Here, the term "control of the temperature of the reaction solution" refers both to varying (raising and lowering) the temperature of the reaction solution and maintaining the temperature of the reaction solution.

In a desirable aspect of the reaction vessel of the present invention, the pressing part is disposed on the cover member so that the contact area between the reaction solution and the reaction chamber can be increased by the pressing of the reaction solution. Here, the term "increasing the contact area between the reaction solution and the reaction chamber" refers to an increase in the contact area between the reaction solution and the reaction chamber compared to the contact area prior to the pressing of the reaction solution; for example, this includes a gradual increase or stepwise increase in the contact area between the reaction solution and the reaction chamber in accordance with the pressing of the reaction solution. In this aspect, the movement of heat via the contact surface between the reaction solution and the reaction chamber can be efficiently accomplished as a result of the contact area between the reaction solution and the reaction chamber being increased by the pressing of the reaction solution; as a result, the temperature of the reaction solution can be controlled more quickly.

In a desirable aspect of the reaction vessel of the present invention, the pressing part is disposed on the cover member so that the contact area between the reaction solution and the pressing part can be increased by the pressing of the reaction solution. Here, the term "increasing the contact area between the reaction solution and the pressing part" includes a gradual or stepwise increase in the contact area between the reaction solution and the pressing part in accordance with the pressing of the reaction solution. In this aspect, the movement of heat via the contact surface between the reaction solution and the pressing part can be efficiently accomplished as a result of the contact area between the reaction solution and the pressing part being increased by the pressing of the reaction solution; as a result, the temperature of the reaction solution can be controlled more quickly.

In a desirable aspect of the reaction vessel of the present invention, the pressing part is disposed on the cover member so that the reaction solution can be formed into a thin configuration by the pressing of the reaction solution. In this aspect, the contact area between the reaction solution and the reaction chamber and the contact area between the reaction solution and the cover member can be increased to an even greater extent by pressing the reaction solution until the reaction solution is formed into a thin configuration. As a result, the movement of heat via the contact surface between the reaction solution and the reaction chamber and the contact surface between the reaction solution and the cover member can be efficiently accomplished, so that the temperature of the reaction solution can be controlled more quickly. Furthermore, in this aspect, control of the temperature of the reaction solution can be accomplished uniformly with respect to the reaction solution as a whole, so that the temperature of the reaction solution can be controlled with good precision. Moreover, in this aspect, most of the surface area of he reaction solution can be formed into a contact surface with the reaction chamber and cover member; as a result, the contact area between the reaction solution and gases such as air or the like that are present inside the reaction chamber can be decreased to a much greater extent, so that the evaporation of the reaction solution into these gases such as air or the like that are present inside the reaction chamber can be suppressed more efficiently.

In a desirable aspect of the reaction vessel of the present invention, the cover member has a first sealing part that can form a tight seal with the circumferential portion of the opening part of the reaction chamber. In this aspect, the first sealing part of the cover member and the circumferential portion of the opening part of the reaction chamber are tightly sealed in a state in which the cover member is mounted on the reaction vessel main body, so that the reaction chamber is tightly closed. As a result, contamination of the reaction solution in the state in which the cover member is mounted on the reaction vessel main body can be prevented.

In a desirable aspect of the reaction vessel of the present invention, the cover member has a second sealing part that can form a tight seal with the inside surface of the reaction chamber. In this aspect, the second sealing part of the cover member and the inside surface of the reaction chamber are tightly sealed during the process of the mounting of the cover member on the reaction vessel main body and/or in the state in which the cover member has been mounted on the reaction vessel main body, so that the reaction chamber is tightly closed. As a result, contamination of the reaction solution during the process of the mounting of the cover member on the reaction vessel main body and/or in the state in which the cover member has been mounted on the reaction vessel main body can be prevented. Furthermore, the reaction solution can be prevented from being pushed out of the reaction chamber via the opening part of the reaction chamber by pressing during the process of the mounting of the cover member on the reaction vessel main body and/or in the state in which the cover member has been mounted on the reaction vessel main body.

In a desirable aspect of the reaction vessel of the present invention, the cover member has a lifting part that makes it possible to lift the cover member. In this aspect, the cover member mounted on the reaction vessel main body can easily be removed from the reaction vessel main body by lifting the lifting part. For example, the removal of the cover member from the reaction vessel main body can be performed after the desired reaction has been caused to proceed inside the reaction chamber; as a result, the reaction product produced by the desired reaction can be recovered.

In a desirable aspect of the reaction vessel of the present invention, the reaction vessel further comprises a heat-conducting metal block which is installed so as to contact the reaction vessel main body and/or the cover member. In this aspect, temperature control of the reaction vessel main body is performed via the contact surface between the reaction vessel main body and the heat-conducting metal block, and temperature control of the cover member is performed via the contact surface between the cover member and the heat-conducting metal block. Furthermore, temperature control of the reaction solution is performed via the contact surface between the reaction solution and the reaction vessel main body and the contact surface between the reaction solution and the cover member. The heat-conducting metal block may be disposed so that this block contacts either the reaction vessel main body or the cover member, or so that this block contacts both the reaction vessel main body and the cover member. Since the heat-conducting metal block can easily be formed in accordance with the shapes of the reaction vessel main body and cover member, the contact area between the reaction vessel main body and the heat-conducting metal block and the contact area between the cover member and the heat-conducting metal block can be increased; as a result, the movement of heat via the heat-conducting metal block can be efficiently accomplished, so that the temperature of the reaction vessel main body and cover member can be quickly controlled. In addition to being used as a medium for the movement of heat (heat exchanger), the heat-conducting metal block can also be used as a member that holds the reaction vessel main body, or as a member that presses the cover member when the cover member is mounted on the reaction vessel main body.

In a desirable aspect of the reaction vessel of the present invention, the reaction solution is a PCR reaction solution, and the reaction vessel is a PCR reaction vessel. In this aspect, the reaction that is caused to proceed inside the reaction chamber is a PCR. In the case of a PCR, the temperature of the reaction solution must be controlled periodically or over time; since the reaction vessel of the present invention makes it possible to achieve quick control of the temperature of the reaction solution, the time required for such a PCR can be shortened by using the reaction vessel of the present invention as a PCR reaction vessel. Furthermore, since this PCR is a technique for amplifying extremely small amounts of template DNA, contamination by other DNA is a serious problem; however, since the reaction vessel of the present invention can prevent contamination of the reaction solution, a PCR can be performed with good precision by using the reaction vessel of the present invention as a PCR reaction vessel. Moreover, since the reaction vessel of the present invention can suppress evaporation of the accommodated reaction solution, a PCR can be caused to proceed even when the amount of the PCR reaction solution is extremely small by using the reaction vessel of the present invention as a PCR reaction vessel.

(2) In order to achieve the abovementioned second object, the present invention provides a reaction apparatus comprising the reaction vessel of the present invention and a temperature control device, wherein the temperature control device is disposed so that the temperatures of the reaction chamber and the cover member can be controlled.

In the reaction apparatus of the present invention, the temperature of the reaction solution can be quickly controlled via the contact surface between the reaction solution and the reaction chamber and the contact surface between the reaction solution and the cover member by controlling the temperatures of the reaction chamber and cover member by means of the temperature control device. For example, the temperature control of the reaction chamber and cover member by means of the temperature control device can be accomplished via the contact surface between the temperature control device and the reaction chamber and the contact surface between the temperature control device and the cover member. Furthermore, in cases where a heat-conducting metal block is provided so that this heat-conducting metal block contacts the reaction vessel main body and/or cover member in the reaction vessel of the present invention, temperature control of the reaction chamber and/or cover member can be accomplished via this heat-conducting metal block.

In the reaction apparatus of the present invention, the temperature of the overall space in which the reaction solution is present can be controlled by controlling the temperatures of the cover member and reaction vessel main body that form the space in which the reaction solution is present. As a result, even if the reaction solution evaporates into the air that is present inside the reaction chamber, the evaporated reaction solution can be liquefied so that progressive evaporation of the reaction solution can be prevented; accordingly, the reaction can be caused to proceed even if the amount of reaction solution is an extremely small amount.

In a desirable aspect of the reaction apparatus of the present invention, the reaction apparatus further comprises a cover member detachment device which can remove the cover member mounted on the reaction vessel main body from the reaction vessel main body. In this aspect, the cover member mounted on the reaction vessel main body can easily be removed from the reaction vessel main body by means of the cover detachment device. In cases where the cover member of the reaction vessel of the present invention has a lifting part that can lift the cover member, the cover member detachment device can be mounted on this lifting part. For example, removal of the cover member from the reaction vessel main body is performed after the desired reaction has been caused to proceed inside the reaction chamber; as a result, the reaction product produced by the desired reaction can be recovered.

In a desirable aspect of the reaction apparatus of the present invention, the reaction vessel is a PCR reaction vessel, and the reaction apparatus is a PCR reaction apparatus. In this aspect, the reaction that is caused to proceed inside the reaction chamber is a PCR. In the case of a PCR, the temperature of the reaction solution must be controlled over time or periodically; since the reaction apparatus of the present invention makes it possible to achieve quick control of the temperature of the reaction solution, the time required for such a PCR can be shortened by using the reaction apparatus of the present invention as a PCR reaction apparatus.

(3) In order to achieve the abovementioned third object, the present invention provides a reaction solution temperature control method comprising a step (a) in which a reaction solution accommodated in a reaction chamber is pressed by a pressing member, and a step (b) in which the temperature of the reaction solution is controlled via the contact surface between the reaction solution and the reaction chamber and the contact surface between the reaction solution and the pressing member.

In step (a) of the temperature control method of the present invention, the reaction solution accommodated in the reaction chamber acquires a contact surface with the reaction chamber and a contact surface with the pressing member as a result of the reaction solution accommodate in the reaction chamber being pressed by the pressing member. As a result, not only the movement of heat via the contact surface between the reaction solution and the reaction chamber, but also the movement of heat via the contact surface between the reaction solution and the cover member, is possible. Accordingly, in step (b), the temperature of the reaction solution can be controlled more quickly than is possible before the pressing of the reaction solution by controlling the temperature of the reaction solution via the contact surface between the reaction solution and the reaction chamber and the contact surface between the reaction solution and the pressing member. As long as the reaction solution has a contact surface with the pressing member, the temperature control of the reaction solution in step (b) may be performed while pressing the reaction solution with the pressing member, or after the reaction solution has been pressed by the pressing member.

For example, the temperature control method of the present invention may be performed using the reaction vessel of the present invention or the reaction apparatus of the present invention; however, the temperature control method of the present invention may also be performed using a reaction vessel or reaction apparatus other than the reaction vessel of the present invention or reaction apparatus of the present invention.

In a desirable aspect of the temperature control method of the present invention, the reaction solution is pressed in the step (a) so that the contact area between the reaction solution and the reaction chamber is increased. In this aspect, the pressing of the reaction solution in step (a) is performed so that the contact area between the reaction solution and the reaction chamber is increased compared to that in the state prior to the pressing of the reaction solution. For example, the pressing of the reaction solution in step (a) can be performed so that the contact area between the reaction solution and the reaction chamber increases gradually or stepwise as the reaction solution is pressed. In this aspect, the movement of heat via the contact surface between the reaction solution and the reaction chamber can be accomplished efficiently by pressing the reaction solution so that the contact area between the reaction solution and the reaction chamber is increased, thus making it possible to control the temperature of the reaction solution more quickly.

In a desirable aspect of the temperature control method of the present invention, the reaction solution is pressed in the step (a) so that the contact area between the reaction solution and the pressing member is increased. In this aspect, for example, the pressing of the reaction solution in step (a) is performed so that the contact area between the reaction solution and the pressing member increases gradually or stepwise as the reaction solution is pressed. In this aspect, the movement of heat via the contact surface between the reaction solution and the pressing member can be accomplished efficiently by pressing the reaction solution so that the contact area between the reaction solution and the pressing member increases, thus making it possible to control the temperature of the reaction solution more quickly.

In a desirable aspect of the temperature control method of the present invention, the reaction solution is pressed in the step (a) so that the reaction solution assumes a thin configuration. In this aspect, the contact area between the reaction solution and the reaction chamber and the contact area between the reaction solution and the pressing member can be increased to a much greater extent by pressing the reaction solution so that the reaction solution assumes a thin configuration. As a result, the movement of heat via the contact surface between the reaction solution and the reaction chamber and the contact surface between the reaction solution and the pressing member can be accomplished efficiently, so that the temperature of the reaction solution can be controlled more quickly. Furthermore, in this aspect, temperature control of the reaction solution can be accomplished uniformly with respect to the reaction solution as a whole, so that the temperature of the reaction solution can be controlled with good precision. Moreover, in this aspect, most of the surface area of the reaction solution can be formed into a contact surface with the reaction chamber and pressing member; as a result, the contact area between the reaction solution and gases such as air or the like that are present inside the reaction chamber can be reduced to a much greater extent, so that the evaporation of the reaction solution into these gases such as air or the like that are present inside the reaction chamber can be suppressed more efficiently.

In a desirable aspect of the temperature control method of the present invention, the reaction solution is a PCR reaction solution. In this aspect, the reaction that is caused to proceed inside the reaction chamber is a PCR. In the case of a PCR, the temperature of the reaction solution must be controlled over time or periodically; since the temperature control method of the present invention makes it possible to achieve quick control of the temperature of the reaction solution, the time required for such a PCR can be shortened by using the temperature control method of the present invention.

### BRIEF DESCRIPTION OF THE DRAWINGS

Fig. 1 is a sectional view of the reaction vessel in one embodiment of the present invention;
Fig. 2 is a plan view of the reaction vessel main body in one embodiment of the present invention;
Fig. 3 is a bottom view of the cover member in one embodiment of the present invention;
Figs. 4 (a) and 4 (b) are respective sectional views of the reaction vessel main body in other embodiments of the present invention;
Figs. 5 (a) through 5 (d) are respective sectional views of the reaction vessel main body in other embodiments of the present invention;
Fig. 6 is a plan view of the reaction vessel main body in other embodiment of the present invention;
Figs. 7 (a) and 7 (b) are respective sectional views of the reaction vessel in other embodiments of the present invention;
Fig. 8 is a bottom view of the cover member in other embodiment of the present invention;
Fig. 9 is a sectional view of the cover member in other embodiment of the present invention;
Fig. 10 is a sectional view of the reaction vessel main body in other embodiment of the present invention;
Fig. 11 is an explanatory diagram showing the state that results when the cover member is removed from the reaction vessel main body by a cover member detachment device comprising a cover member detachment part and a reaction vessel main body fastening part;
Fig. 12 is a sectional view of the reaction vessel in other embodiment of the present invention;
Fig. 13 is an explanatory diagram showing changes in the state of the reaction solution during the process of the mounting of the cover member on the reaction vessel main body; and
Fig. 14 is a sectional view showing a state in which a Peltier element is mounted on the reaction vessel in one embodiment of the present invention.

### BEST MODE FOR CARRYING OUT THE INVENTION

Below, embodiments of the reaction vessel of the present invention will be described with reference to the attached figures.

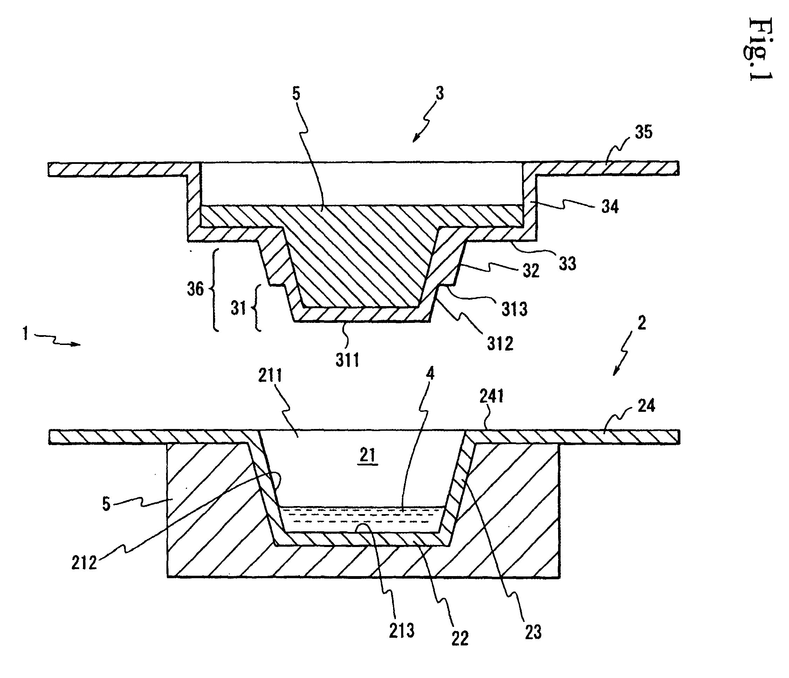

Fig. 1 is a sectional view showing one embodiment of the reaction vessel of the present invention.

As is shown in Fig. 1, the reaction vessel 1 of the present embodiment comprises a reaction vessel main body 2, a cover member 3, and a heat-conducting metal block 5.

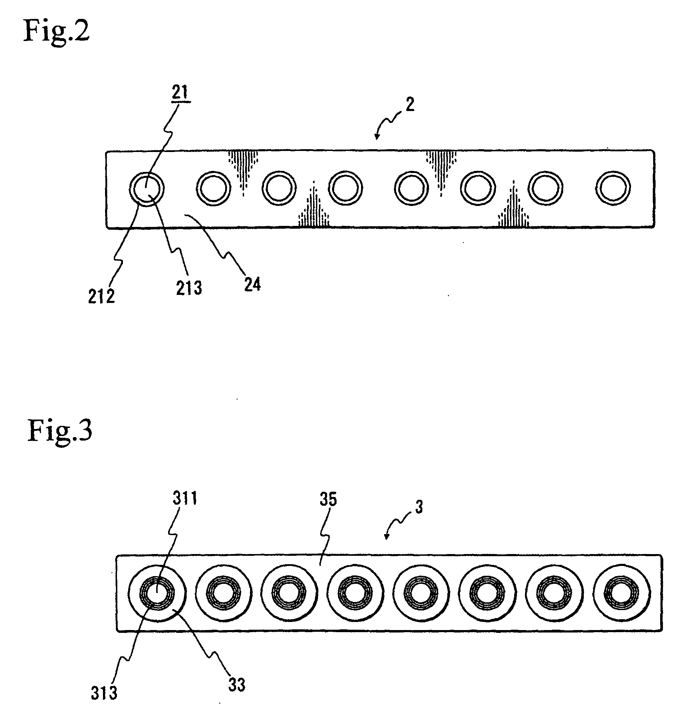

Fig. 2 is a plan view of the reaction vessel main body 2 in the present embodiment, and Fig. 3 is a bottom view of the cover member 3 in the present embodiment.

As is shown in Figs. 1 and 2, the reaction vessel main body 2 is constructed from a circular bottom plate part 22, a tubular part 23 which is installed in an upright position so that this tubular part 23 gradually increases in diameter in the upward direction from the circumferential edge of the bottom plate part 22, and a flat-plate part 24 which is disposed on the upper end of the tubular part 23.

As is shown in Figs. 1 and 2, the reaction vessel main body 2 comprises a reaction chamber 21 which has an opening part 211 in the upper end, and which can accommodate a reaction solution 4. As is shown in Figs. 1 and 2, the opening part 211 of the reaction chamber 21 is formed in the upper surface of the flat-plate part 24 of the reaction vessel main body 2, and a space that allows the reaction chamber 21 to accommodate the reaction solution 4 is formed by the bottom plate part 22 and tubular part 23 of the reaction vessel main body 2, with the reaction chamber 21 being formed in the reaction vessel main body 2 as a recessed part which has an opening part 211 in the upper end.

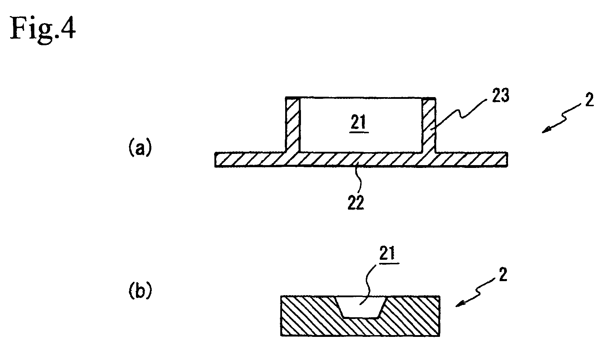

The manner in which the reaction chamber 21 in the reaction vessel main body 2 is formed may be varied as long as the reaction chamber 21 has an opening part 211 in the upper end, and can accommodate the reaction solution 4. For example, as is shown in Fig. 4 (a), a recessed part formed by the upright installation of a circular-tube type or square-tube type tubular part 23 on the upper surface of a circular or rectangular bottom plate part 22 may be used as the reaction chamber 21, or, as is shown in Fig. 4 (b), a recessed part formed as a hole that is recessed into the interior of the reaction vessel main body 2 may be used as the reaction chamber 21.

As is shown in Figs. 1 and 2, the opening part 211 of the reaction chamber 21 has a circular shape, and the side surfaces 212 of the reaction chamber 21 have a tubular shape that shows a gradual decrease in diameter in the downward direction from the opening part 211, and the bottom surface 213 of the reaction chamber 21 is formed as a circular planar surface. Accordingly, in longitudinal section, the structure of the reaction chamber 21 has a trapezoidal shape that is recessed so that the diameter gradually decreases in the downward direction from the opening part 211.

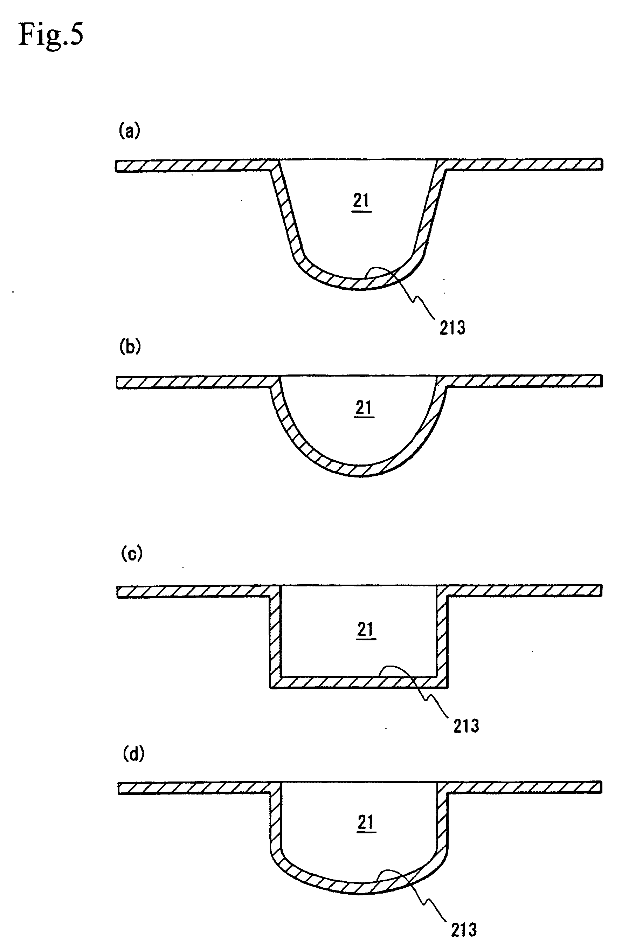

The structure of the reaction chamber 21 may be altered as long as the reaction chamber 21 has an opening part 211 in the upper end and can accommodate the reaction solution 4. For example, the shape of the opening part 211 of the reaction chamber 21 or the shape of the bottom surface 213 may be formed as a rectangular shape, or the bottom surface 213 of the reaction chamber 21 may be formed as a curved surface as shown in Fig. 5 (a). Furthermore, the structure of the reaction chamber 21 may be formed with a semicircular shape in longitudinal section that is recessed so that the diameter gradually decreases in the downward direction from the opening part 211 as shown in Fig. 5 (b), or may be formed with a rectangular shape in longitudinal section that is recessed so that the same diameter is maintained in the downward direction from the opening part 211 as shown in Fig. 5 (c). Moreover, the bottom surface 213 of the reaction chamber 21 shown in Fig. 5 (c) may also be formed as a curved surface as shown in Fig. 5 (d) .

As is shown in Figs. 1 and 2, the structure of the reaction chamber 21 is a structure in which the area of the opening part 211 is larger than the area of the bottom surface 213, so that the reaction solution 4 added from the opening part 211 can easily reach the bottom surface 213 "as is" (even if no force other than gravity is applied to the reaction solution 4 in the downward direction). Depending on the manner in which the reaction solution 4 is added, there may be cases in which the reaction solution 4 adheres to the side surfaces 212 of the reaction chamber 21; in such cases, however, the reaction solution 4 can be caused to reach the bottom surface 213 of the reaction chamber 21 by utilizing a vortex mixer or the like to apply a vibration to the reaction vessel main body 2.

There are no particular restrictions on the diameter of the opening part 211 of the reaction chamber 21; preferably, however, this diameter is 4 to 5 mm. There are no particular restrictions on the depth of the reaction chamber 21, either; preferably, however, this depth is 3 to 5 mm. Likewise, there are no particular restrictions on the diameter of the bottom surface 213 of the reaction chamber 21; preferably, however, this diameter is 2 to 3 mm.

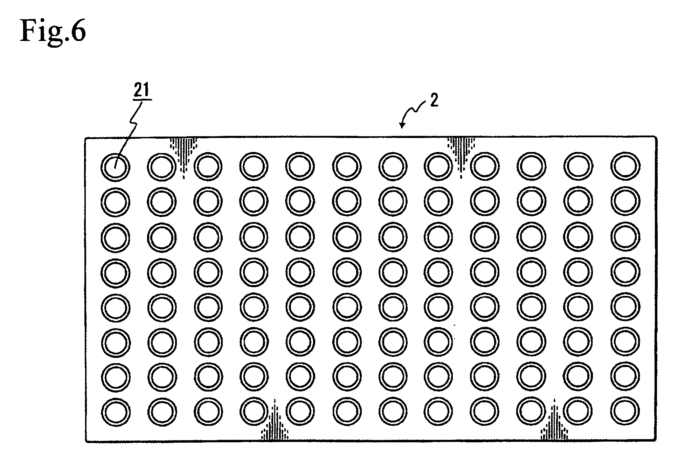

As is shown in Fig. 2, the reaction vessel main body 2 comprises eight reaction chambers 21 that are lined up in a single row. The number and positions of the reaction chambers 21 comprised by the reaction vessel main body 2 may be altered. For example, it would also be possible to install 8 longitudinal rows × 12 lateral rows, for a total of 96 reaction chambers 21, in the reaction vessel main body 2 as shown in Fig. 6. The number of reaction chambers comprised by the reaction vessel main body 2 may also be a single reaction chamber; however, from the standpoint of sample treatment efficiency, it is desirable that there be a plurality of reaction chambers. Since sample injection devices that mount an eight-head nozzle unit are commercially marketed, it is desirable that the reaction vessel main body 2 comprise 8 reaction chambers 21 in a single row as shown in Figs. 2 and 6 in cases where the injection of the reaction solution into the reaction chambers 21 is automated.

There are no particular restrictions on the size of the reaction vessel main body 2; this size may be appropriately determined in accordance with the number of reaction chambers 21 and the like.

There are no particular restrictions on the material of the reaction vessel main body 2, as long as this material is not corroded by the reaction solution 4, and can withstand the conditions (e. g., reaction temperature) of the reaction performed in the reaction chambers 21.

Examples of materials that can be used as the material of the reaction vessel main body 2 include thermoplastic resins, metals, glass and the like. If a thermoplastic resin is used as the material of the reaction vessel main body 2, then the reaction vessel main body 2 can easily be molded by ordinary methods such as injection molding or the like. In cases where the reaction temperature reaches a high temperature (e. g., 90 to 100°C), it is desirable that a material that is superior in terms of heat resistance, e.g., engineering plastics (for example, polyamides, polyacetals, polycarbonates, polyesters or the like), be used.

The side surfaces 212 and bottom surfaces 213 of the reaction chambers 21 may be appropriately treated in accordance with the type of reaction solution 4 involved. For example, in cases where enzymes and DNA are contained in the reaction solution 4, the adhesion of such enzymes or DNA to the side surfaces 212 and bottom surface 213 of the reaction chambers 21 can be prevented by subjecting the inside surfaces of the reaction chambers 21 to a finishing treatment such as a siliconizing treatment or the like.

As is shown in Fig. 1, the bottom plate part 22, tubular part 23 and flat-plate part 24 that constitute the reaction vessel main body 2 have a more or less uniform thickness; however, the thicknesses of the bottom plate part 22, tubular part 23 and flat-plate part 24 may be varied. From the standpoint of quick control of the temperature of the reaction solution 4 accommodated in the reaction chamber 21, it is desirable that the thicknesses of the bottom plate part 22 and tubular part 23 be on the thin side. The thicknesses of the bottom plate part 22, tubular part 23 and flat-plate part 24 are preferably 0.1 to 0.5 mm.

As is shown in Figs. 1 and 3, the cover member 3 is constructed from protruding parts 36 that protrude downward, doughnut-plate-form first sealing parts 33 that are disposed on the upper ends of the protruding parts 36, tubular parts 34 that are disposed in upright positions on the circumferential edges of the first sealing parts 33, and flat-plate parts 35 that are disposed on the upper ends of the tubular parts 34.

As is shown in Fig. 3, eight protruding parts 36 are disposed on the cover member 3 in positions corresponding to the reaction chambers 21 comprised by the reaction vessel main body 2 (see Fig. 2). The number and positions of the protruding parts 36 on the cover member 3 may be altered in accordance with the number and positions of the reaction chambers 21 comprised by the reaction vessel main body 2.

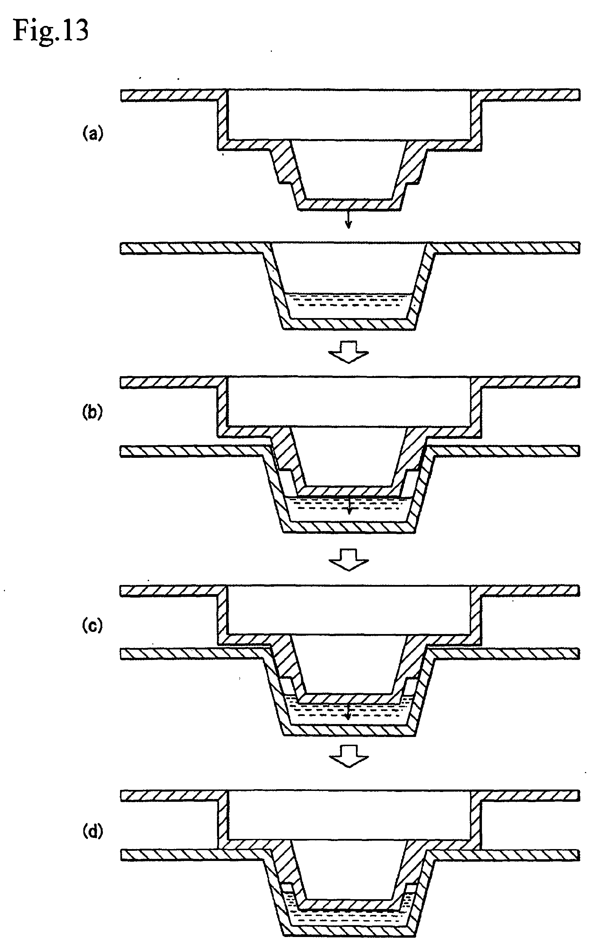

The protruding parts 36 are disposed on the cover member 3 so that these protruding parts 36 engage with the recessed parts formed as reaction chambers 21 in the reaction vessel main body 2, and are arranged so that when the cover member 3 is mounted on the reaction vessel main body 2, the protruding parts 36 engage with the recessed parts formed as reaction chambers 21 in the reaction vessel main body 2, thus sealing the opening parts 211 of the reaction chambers 21 (see Fig. 13).

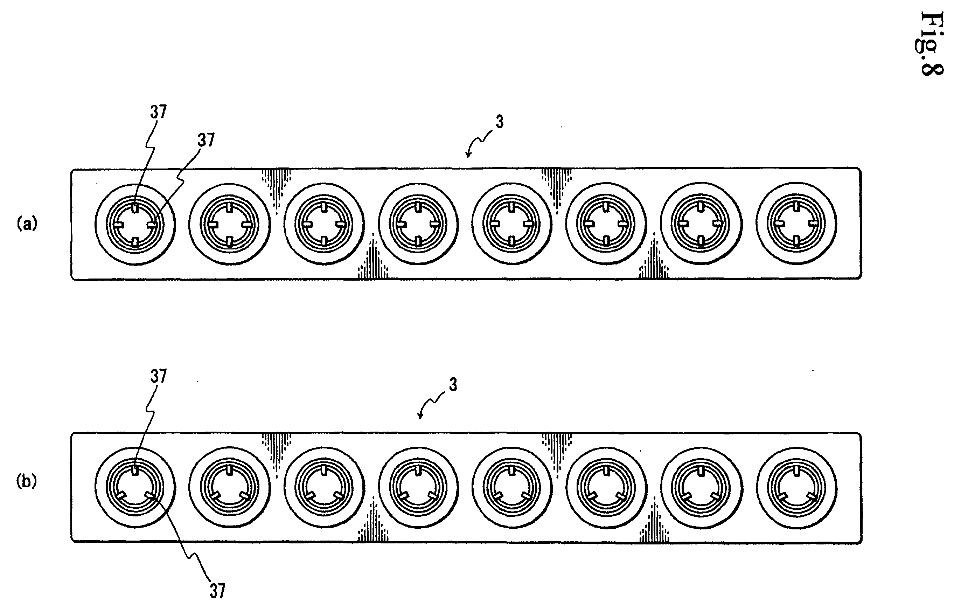

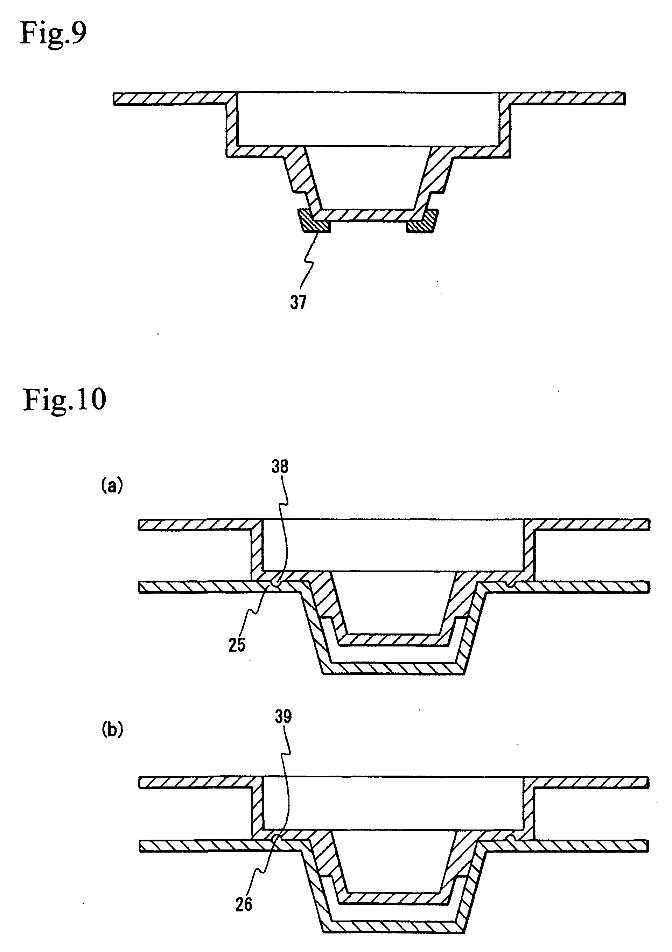

The protruding parts 36 are disposed on the cover member 3 so that in a state in which the protruding parts 36 engage with the recessed parts formed as reaction chambers 21 in the reaction vessel main body 2, the tip end portions of the protruding parts 36 (first contact parts 311 of the pressing parts 31) do not contact the bottom surfaces 213 of the reaction chambers 21 (see Fig. 13). The reason for this is that if the tip end portions of the protruding parts 36 and the bottom surfaces 213 of the reaction chambers 21 contact each other, the contact area between the reaction solution 4 and the tip end portions of the protruding parts 36 and the contact area between the reaction solution 4 and the bottom surfaces 213 of the reaction chambers 21 will be reduced, so that temperature control of the reaction solution 4 via these contact surfaces becomes difficult. From the standpoint of preventing contact between the protruding parts 36 and the bottom surfaces 213 of the reaction chambers 21, it is desirable to form ribs 37 on the lower portions of the protruding parts 36 as shown in Figs. 8 and 9.

As is shown in Fig. 1, each protruding part 36 is constructed from a pressing part 31 and a second sealing part 32.

As is shown in Figs. 1 and 2, each pressing part 31 is constructed from a first contact part 311, a second contact part 312, and a third contact part 313. As is shown in Figs. 1 and 2, the first contact part 311 has a disk shape, and is disposed so that this contact part 311 faces the bottom surface 213 of the reaction chamber 21 in a state in which the cover member 3 is mounted on the reaction vessel main body 2. As is shown in Figs. 1 and 2, the second contact part 312 has a tubular shape which is disposed in an upright attitude so that the diameter gradually increases from the circumferential edge of the first contact part 311, and this second contact part 312 is disposed so as to face the side surfaces 212 of the reaction chamber 21 in a state in which the cover member 3 is mounted on the reaction vessel main body 2. As is shown in Figs. 1 and 2, the third contact part 313 has a doughnut shape which is disposed on the upper end of the second contact part 312, and this third contact part 313 is disposed so as to face the bottom surface 213 of the reaction chamber 21 in a state in which the cover member 3 is mounted on the reaction vessel main body 2.

In the process of the mounting of the cover member 3 on the reaction vessel main body 2, the reaction solution 4 accommodated in the reaction chambers 21 first contacts the first contact parts 311, then contacts the second contact parts 312, and finally contacts the third contact parts 313 (see Fig. 13). Specifically, the pressing parts 31 are disposed on the cover member 3 so that these pressing parts 31 press the reaction solution 4 while increasing the contact area with the reaction solution 4. Depending on the amount of reaction solution 4 accommodated in the reaction chambers 21, there may be cases in which the reaction solution 4 and the second contact parts 312 and third contact parts 313 do not contact each other, or cases in which the reaction solution 4 and the third contact parts 313 do not contact each other. However, since a greater contact area between the reaction solution 4 and the pressing parts 31 allows quicker temperature control of the reaction solution 4 via the contact surfaces between the reaction solution 4 and the pressing parts 31, it is desirable from the standpoint of quick temperature control of the reaction solution 4 that the pressing parts 31 be disposed on the cover member 3 so that the reaction solution 4 and second contact parts 312 contact each other, and it is even more desirable that the pressing parts 31 be disposed on the cover member 3 so that the reaction solution 4 and the second contact parts 312 and third contact parts 313 contact each other. Furthermore, in cases where the reaction solution 4 and third contact parts 313 contact each other, the contact area between the reaction solution 4 and gases such as air or the like present inside the reaction chambers 21 can be reduced, so that evaporation of the reaction solution 4 can be suppressed. Accordingly, from the standpoint of suppression of the evaporation of the reaction solution 4, it is desirable that the pressing parts 31 be disposed on the cover member 3 so that the reaction solution 4 and third contact parts 313 contact each other.

The pressing parts 31 are disposed on the cover member 3 so that the reaction solution 4 accommodated in the reaction chambers 21 can be pressed in the process of the mounting of the cover member 3 on the reaction vessel main body 2. In the process of the mounting of the cover member 3 on the reaction vessel main body 2, the pressing parts 31 advance into the interiors of the reaction chambers 21, contact the reaction solution 4 accommodated inside the reaction chambers 21, and press the reaction solution 4 (see Fig. 13). The reaction solution 4 accommodated in the reaction chambers 21 can be pressed by the pressing parts 31 in the process of the mounting of the cover member 3 on the reaction vessel main body 2 by adjusting the structure, size and the like of the pressing parts 31 in accordance with the structure, size and the like of the reaction chambers 21.

The pressing parts 31 are disposed on the cover member 3 so that the reaction solution 4 can be formed into a thin configuration in a state in which the cover member 3 is mounted on the reaction vessel main body 2. Specifically, the pressing parts 31 are disposed on the cover member 3 so that the distance between the first contact parts 311 of the pressing parts 31 and the bottom surfaces 213 of the reaction chambers 21 and the distance between the second contact parts 312 of the pressing parts 31 and the side surfaces 212 of the reaction chambers 21 are shortened in a state in which the cover member 3 is mounted on the reaction vessel main body 2. As a result, the reaction solution 4 is present in a thin configuration (or film-form configuration) between the first contact parts 311 of the pressing parts 31 and the bottom surfaces 213 of the reaction chambers 21 and between the second contact part s 312 of the pressing parts 31 and the side surfaces 212 of the reaction chambers 21 (see Fig. 13). The distance between the first contact parts 311 and the bottom surfaces 213 of the reaction chambers 21 and the distances between the second contact parts 312 and the side surfaces 212 of the reaction chambers 21 (specifically, these distances correspond to the thickness of the thin reaction solution 4) are preferably in the range of 0.1 to 0.5 mm; furthermore, it is even more desirable that the distance between the first contact parts 311 and the bottom surfaces 213 of the reaction chambers 21 and the distances between the second contact parts 312 and the side surfaces 212 of the reaction chambers 21 (specifically, these distances correspond to the thickness of the thin reaction solution 4) be uniform.

The structure of the pressing parts 31 can be varies as long as these pressing parts 31 can press the reaction solution 4 accommodated in the reaction chambers 21. For example, the first contact parts 311 of the pressing parts 31 may be formed with a rectangular plate-form shape, the undersurfaces of the first contact parts 311 may be formed as curved surfaces, or the second contact parts 312 may be formed with a cylindrical shape or square tubular shape that maintains the same diameter in the upward direction from the circumferential edges of the first contact parts 311.

Ribs 37 may also be formed on the lower portions of the pressing parts 31. For example, four ribs 37 that are arranged in a cruciform configuration may be formed in the area extending from the circumferential edge portions of the first contact parts 311 to the lower portions of the second contact parts 312 a shown in Figs. 8 (a) and 9. These ribs 37 act to prevent contact between the protruding parts 36 and the bottom surfaces 213 of the reaction chambers 21, and also act as spacers that form the spaces in which the reaction solution 4 is present. Furthermore, the ribs 37 also act to determine the distance between the first contact parts 311 of the pressing parts 31 and the bottom surfaces 213 of the reaction chambers 21 and the distance between the second contact parts 312 of the pressing parts 31 and the side surfaces 212 of the reaction chambers 21. The size of the ribs 37 is preferably a size that makes it possible to form the reaction solution 4 into a thin configuration. It is desirable that the ribs 37 be disposed so that these ribs 37 do not divide the reaction solution 4. The reason for this is that if the reaction solution 4 is divided, the efficiency of the reaction that takes place inside the reaction chambers 21 drops.

The number, shape, structure, positions and the like of the ribs 37 may be varied. For example, the number of ribs 37 may be set at three as shown in Fig. 8 (b), or the ribs may be disposed only on the first contact parts 311 or second contact parts 312 of the pressing parts 31. Furthermore, such ribs 37 may also be disposed on either the bottom surfaces 213 or side surfaces 212 of the reaction chambers 21, or on both of these surfaces. Alternatively, such ribs 37 may be omitted altogether.

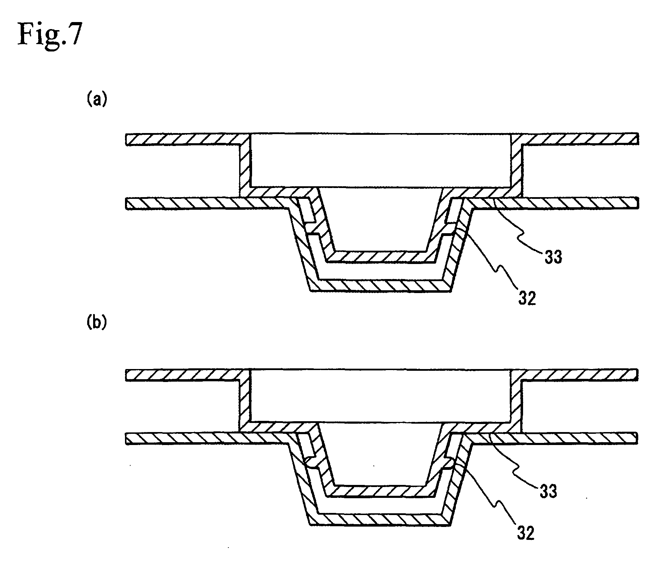

As is shown in Figs. 1 and 2, the second sealing parts 32 are formed with a tubular shape such that the diameter of the second sealing parts 32 gradually increases along the upward direction from the circumferential edges of the third contact parts 313 of the pressing parts 31, and are continuous with the first sealing parts 33 at the top ends thereof. The second sealing parts 32 are disposed so that these sealing parts 32 can form a tight seal with the side surfaces 212 of the reaction chambers 21 during the process of the mounting of the cover member 3 on the reaction vessel main body 2 and in a state in which the cover member 3 has been mounted on the reaction vessel main body 2. The structure, size and the like of the second sealing parts 32 may be varied as long as these sealing parts 32 can form a tight seal with the side surfaces 212 of the reaction chambers 21. For example, the second sealing parts 32 may be disposed so that these parts are not continuous with the first sealing parts 33, as shown in Fig. 7 (a), or may be disposed so that these parts make linear contact with the side surfaces 212 of the reaction chambers 21 instead of surface contact, as shown in Fig. 7 (b).

The first sealing parts 33 are disposed on the cover member 3 so that these parts can form a tight seal with the circumferential portions 241 of the opening parts 211 of the reaction chambers 21 (the portions of the upper surface of the flat-plate part 24 of the reaction vessel main body 2 that constitute the circumferential portions of the opening parts 211 of the reaction chambers 21) in a state where the cover member 3 is attached to and covers the reaction vessel main body 2. The structure, size and the like of the first sealing parts 33 may be varied as long as these parts can form a tight seal with the circumferential portions 241 of the opening parts 211 of the reaction chambers 21. For example, recessed parts 25 can be formed in the circumferential portions 241 of the opening parts 211 of the reaction chambers 21 and protruding parts 38 that can engage with these recessed parts 25 can be formed on the first sealing parts 33 as shown in Fig. 10 (a), or protruding parts 26 can be formed on the circumferential portions 241 of the opening parts 211 of the reaction chambers 21 and recessed parts 39 that can engage with these protruding parts 26 can be formed in the first sealing parts 33 as shown in Fig. 10 (b).

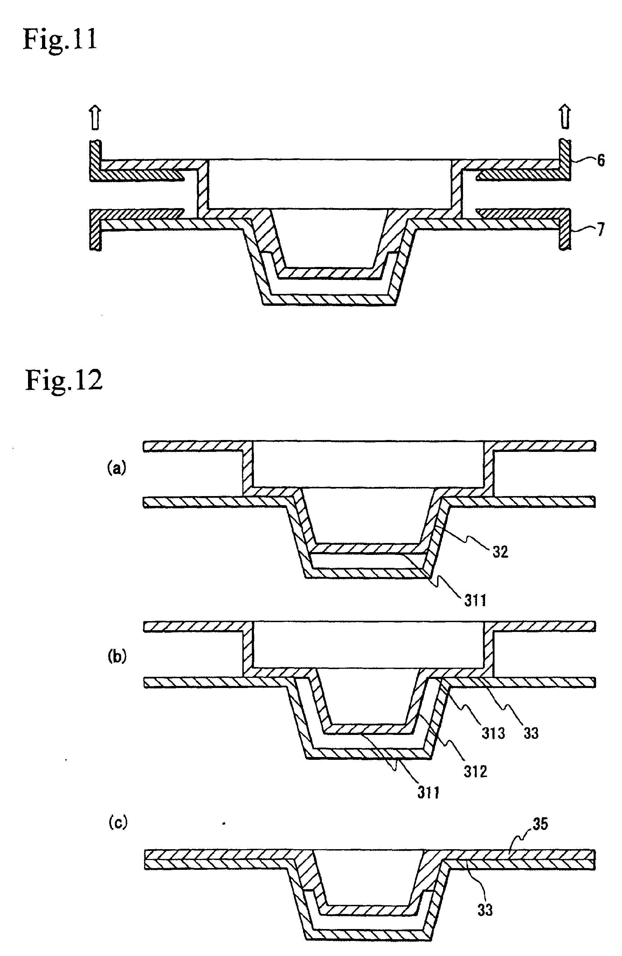

The flat-plate part 35 of the cover member 3 can be used as a lifting part that makes it possible to lift the cover member 3. For example, the cover member 3 mounted on the reaction vessel main body 2 can be removed from the reaction vessel main body 2 by gripping and lifting the flat-plate part 35. Furthermore, as is shown in Fig. 11, it is also possible to remove the cover member 3 mounted on the reaction vessel main body 2 from the reaction vessel main body 2 by lifting the cover member 3 by means of a cover member detachment device comprising a cover member detachment part 6 and a reaction vessel main body fastening part 7. The cover member detachment part 6 of the cover member detachment device is inserted between the flat-plate part 35 of the cover member 3 and the flat-plate part 24 of the reaction vessel main body 2, so that this cover member detachment part 6 contacts the undersurface of the flat-plate part 35 of the cover member 3, and the flat-plate part 35 of the cover member 3 is lifted. The reaction vessel main body fastening part 7 of the cover member detachment device is inserted between the flat-plate part 35 of the cover member 3 and the flat-plate part 24 of the reaction vessel main body 2, so that this reaction vessel main body fastening part 7 contacts the upper surface of the flat-plate part 24 of the reaction vessel main body 2, thus fastening the reaction vessel main body 2 in place. The structure, size and the like of the cover member detachment part 6 and reaction vessel main body fastening part 7 of the cover member detachment device may be alter in accordance with the structure, size and the like of the flat-plate part 35 of the cover member 3 and the flat-plate part 24 of the reaction vessel main body 2.

The structure, size and the like of the flat-plate part 35 may be varied. For example, the flat-plate part 35 may be altered as long as this part can be used as a lifting part that is able to lift the cover member 3, and a structure other than a flat-plate structure may also be used.

A space that is formed by the tubular parts 34 of the cover member 3 is present between the flat-plate part 35 of the cover member 3 and the flat-plate part 24 of the reaction vessel main body 2; as result of the presence of this space, the gripping of the flat-plate part 35 of the cover member 3 is facilitated when the cover member 3 is lifted; furthermore, the insertion of the cover member detachment part 6 and reaction vessel main body fastening part 7 of the cover member detachment device is facilitated. The structure, size and the like of the tubular parts 34 may be varied.

The structure of the cover member 3 may be varied as long as the cover member 3 can seal the opening parts 211 of the reaction chambers 21, and a s long as the cover member 3 has pressing parts 31 that can press the reaction solution 4 accommodated in the reaction chambers 21.

For example, as is shown in Fig. 12 (a), it would also be possible to connect the first contact parts 311 and second sealing parts 32 as continuous parts without forming second contact parts 312 or third contact parts 313 on the pressing parts 31 of the cover member 3. However, from the standpoint of increasing the contact area between the reaction solution 4 and the pressing parts 31, it is desirable to form second contact parts 312 and third contact parts 313 on the pressing parts 31.

Furthermore, as is shown in Fig. 12 (b), it would also be possible to connect the third contact parts 313 and first sealing parts 33 as continuous parts without forming second sealing parts 32 on the cover member 3. However, from the standpoint of increasing the degree of sealing of the reaction chambers 4, it is desirable to form second sealing parts 32.

Furthermore, as is shown in Fig. 12 (c), it would also be possible to connect the first sealing parts 33 and flat-plate part 35 as continuous parts without forming tubular parts 34 on the cover member 3. However, from the standpoint of increasing the ease of lifting in cases where the flat-plate part 35 of the cover member 3 is used as a lifting part, it is desirable to form tubular parts 34.

There are no particular restrictions on the material of the cover member 3 as long as this material is not corroded by the reaction solution 4, and can withstand the conditions (e. g., reaction temperature) of the reaction that occurs inside the reaction chambers 21. Examples of materials that can be used as the material of the cover member 3 include plastics, metal, glass and the like. If a thermoplastic resin is used as the material of the cover member 3, then the cover member 3 can easily be formed by ordinary methods such as injection molding or the like. In cases where the reaction temperature reaches a high temperature (e. g., 90 to 100°C), it is desirable to use a material that is superior in terms of heat resistance, e. g., engineering plastics (for example, polyamides, polyacetals, polycarbonates, polyesters and the like).

There are no particular restrictions on the size of the cover member 3; this size can be appropriately determined in accordance with the size and the like of the reaction vessel main body 2. There are no particular restrictions on the thickness of the cover member 3; however, from the standpoint of increasing the efficiency of the movement of heat, it is desirable that the cover member 3 have a small thickness. The thickness of the cover member 3 is preferably 0.1 to 0.5 mm.

As is shown in Fig. 1, two heat-conducting metal blocks 5 are installed on the reaction vessel 1. One of these blocks is disposed on the cover member 3, and the other block is disposed on the reaction vessel main body 2.

As is shown in Fig. 1, the heat-conducting metal block 5 disposed on the cover member 3 has a protruding part that protrudes downward, and this protruding part is disposed so as to engage with a recessed part that is formed inside the protruding part 36 of the cover member 3.

Furthermore, as is shown in Fig. 1, the heat-conducting metal block 5 disposed on the reaction vessel main body 2 has a recessed part, and is disposed so that this recessed part engages with a protruding part which is formed by the bottom plate part 22 and tubular part 23 of the reaction vessel main body 2, and which protrudes downward.

As a result of a temperature control device being mounted on the heat-conducting metal blocks 5, the temperatures of the reaction vessel main body 2 and cover member 3 can be controlled via the contact surface between the heat-conducting metal block 5 and the reaction vessel main body 2 and the contact surface between the heat-conducting metal block 5 and the cover member 3. Furthermore, by controlling the temperatures of the reaction vessel main body 2 and cover member 3, it is possible to control the temperature of the reaction solution 4 via the contact surface between the reaction solution 4 and the reaction vessel main body 2 and the contact surface between the reaction solution 4 and the cover member 3.

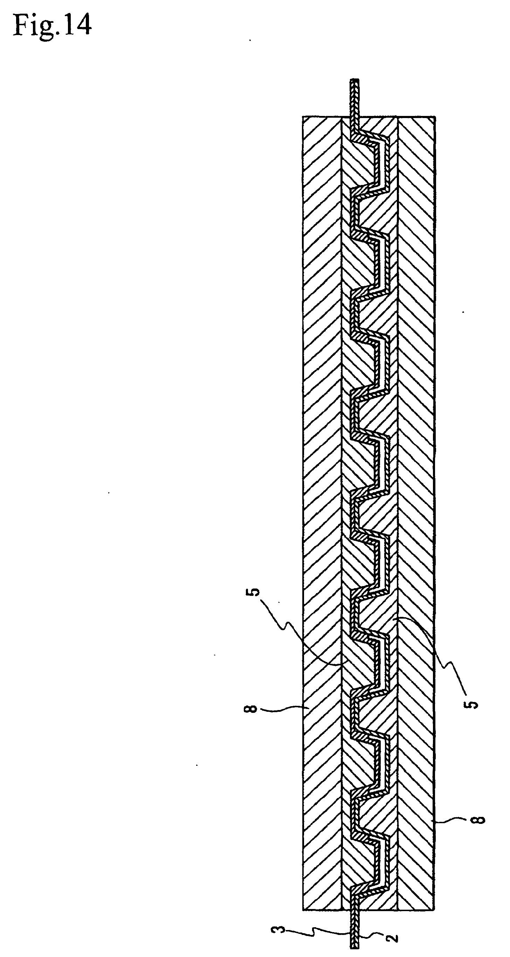

An ordinary commercially marketed device can be used as the temperature control device that is mounted on the heat-conducting metal blocks 5. The temperature control device may be installed so that this temperature control device controls the temperatures of the reaction vessel main body 2 and cover member 3, or the temperature control device may be mounted directly on the reaction vessel main body 2 and cover member 3. There are no particular restrictions on the cooling/heating means of the temperature control device; for example, a Peltier element or the like may be used. In cases where a Peltier element is used as the cooling/heating means of the temperature control device, quick temperature control of the reaction vessel main body 2 and cover member 3 can be accomplished by (for example) causing a Peltier element 8 to contact the undersurface of the heat-conducting metal block 5 disposed on the undersurface of the reaction vessel main body 2, and causing a Peltier element 8 to contact the upper surface of the heat-conducting metal block 5 disposed on the upper surface of the cover member 3, as shown in Fig. 14.

The structure, size the like of the heat-conducting metal blocks 5 may be varied. Temperature control of the reaction vessel main body 2 and cover member 3 can be accomplished quickly by increasing the contact area between the heat-conducting metal blocks 5 and the reaction vessel main body 2 and cover member 3. Accordingly, it is desirable that the heat-conducting metal blocks 5 have a structure which is such that the contact area between the heat-conducting metal blocks 5 and the reaction vessel main body 2 and cover member 3 is large. It would also be possible to install a heat-conducting metal block 5 only on the cover member 3, or only on the reaction vessel main body 2. Furthermore, it would also be possible to install no heat-conducting metal block 5 on either the cover member 3 or the reaction vessel main body 2.

By appropriately varying the structure, size and the like of the heat-conducting metal blocks 5, it is possible to use these heat-conducting metal blocks 5 as a holder that supports the reaction vessel main body 2 and as a pressing member that presses the cover member 3 when the cover member 3 is mounted on the reaction vessel main body 2, in addition to using these heat-conducting metal blocks 5 as heat exchangers.

There are no particular restrictions on the material of the heat-conducting metal blocks 5 as long as this material is a metal that possesses thermal conductivity; preferably, however, this material is a metal with good thermal conductivity such as aluminum, copper, iron or the like. Furthermore, the material of the heat-conducting metal blocks 5 may also be an alloy of two or more heat-conducting metals.

Fig. 13 shows diagrams that illustrate changes in the state of the reaction solution 4 that occur in the process of the mounting of the cover member 3 on the reaction vessel main body 2.

Before the cover member 3 is mounted on the reaction vessel main body 2, as is shown in Fig. 13 (a), the pressing part 31 and reaction solution 4 are not in contact with each other, and the reaction solution 4 contacts only the side surfaces 212 and bottom surface 213 of the reaction chamber 21. In this case, the contact area between the reaction solution 4 and side surfaces 212 and bottom surface 213 of the reaction chamber 21 is fixed.

In the process of the mounting of the cover member 3 on the reaction vessel main body 2, the first contact part 311 of the pressing part 31 first contacts the upper surface of the reaction solution 4 as shown in Fig. 13 (b). In this case, there is no change in the contact area between the reaction solution 4 and the side surfaces 212 and bottom surface 213 of the reaction chamber 21.

After the first contact part 311 of the pressing part 31 has contacted the upper surface of the reaction solution 4, the first contact part 311 of the cover member 3 presses the reaction solution 4 as shown in Fig. 13 (c). As a result, the liquid level of the reaction solution 4 rises, so that the second contact part 312 of the pressing part 31 contacts the reaction solution 4 and begins to press the reaction solution 4, and so that the contact area between the reaction solution 4 and the side surfaces 212 of the reaction chamber 21 is increased. When pressing by the first contact part 311 and second contact part 312 is continued, the liquid level of the reaction solution 4 rises even further, and the contact area between the reaction solution 4 and the second contact part 312 and the contact area between the reaction solution 4 and the side surfaces 212 of the reaction chamber 21 are increased even further. Depending on the amount of the reaction solution 4, the third contact part 313 of the pressing part 31 may also contact the reaction solution 4 and press the reaction solution 4. Thus, the pressing part 31 presses the reaction solution 4 while increasing the contact area with the reaction solution 4, and the contact area between the reaction solution 4 and the side surfaces 212 of the reaction chamber 21 increases in accordance with the pressing of the reaction solution 4 by the pressing part 31.

In a case where the third contact part 313 of the pressing part 31 and the reaction solution 4 contact each other, the pressing of the reaction solution 4 by the pressing part 31 may be ended at this point in time, or the pressing of the reaction solution 4 may be continued even further. In cases where the pressing of the reaction solution 4 is continued, the application of pressure to the cover member 3 is necessary. By applying pressure to the cover member 3 and continuing the pressing of the reaction solution 4, it is possible to discharge gases such as air or the like present inside the reaction chamber 21 to the outside of the reaction chamber 21 via the second sealing part 32 and first sealing part 33. In this case, the degree of sealing between the second sealing part 32 and the side surfaces 212 of the reaction chamber 21 and the degree of sealing between the first sealing part 33 and the upper surface of the flat-plate part 24 of the reaction vessel main body 2 are not complete, but are rather degrees of sealing that allow the expulsion of gases such as air or the like present inside the reaction chamber 21 to the outside of the reaction chamber 21 as a result of the application of pressure to the cover member 3. In cases where the reaction vessel main body 2 and cover member 3 are formed from a material that has a certain degree of elasticity such as a plastic or the like, a degree of sealing that allows the expulsion of gases such as air or the like present inside the reaction chamber 21 to the outside of the reaction chamber 21 by the application of pressure to the cover member 3 can be achieved.

After the cover member 3 has been mounted on the reaction vessel main body 2, the reaction solution 4 is present in a thin configuration between the pressing part 31 of the cover member 3 and the side surfaces 212 and bottom surface 213 of the reaction chamber 21 as shown in Fig. 13 (d).

Temperature control of the reaction solution 4 is generally accomplished via the contact surface between the pressing part 31 and the reaction solution 4 and the contact surface between the reaction chamber 21 and the reaction solution 4 after the cover member 3 has been mounted on the reaction vessel main body 2. However, if the first contact part 311 of the pressing part 31 has contacted the upper surface of the reaction solution 4, temperature control of the reaction solution 4 can also be accomplished during the process of the mounting of the cover member 3 on the reaction vessel main body 2.

The reaction solution 4 may be appropriately selected in accordance with the desired reaction that is caused to proceed in the reaction chamber 21. There are no particular restrictions on the reaction that is the object of the reaction vessel 1; however, it is desirable that the reaction vessel 1 be used for reactions in which adjustment of the reaction temperature is necessary when the reaction is caused to proceed. Such adjustment of the reaction temperature may include maintenance of the reaction temperature at a temperature within a fixed range, or variation of the reaction temperature over time or periodically. Examples of reactions that require adjustment of the reaction temperature when the reaction is caused to proceed include enzyme reactions, PCR and the like. Enzymes are proteins and may be denatured by extreme heat; accordingly, enzyme reactions require adjustment of the reaction temperature when the reaction is caused to proceed. Furthermore, in the case of PCR, periodic changes or changes over time to temperatures in three stages, i. e., a temperature at which double-stranded DNA acting as a template is dissociated into single-stranded DNA, a temperature in which oligonucleotide primers are annealed with the dissociated single-stranded DNA, and a temperature at which complementary DNA chains are synthesized from the primer sites by a polymerase, are generally required. The reaction vessel 1 is especially suitable for reactions in which multistage temperature adjustment is required, as in PCR.

In cases where the reaction that is caused to proceed in the reaction chamber 21 is a PCR, the reaction solution 4 is a PCR reaction solution. A PCR reaction solution ordinarily contains H₂O, buffers, MgCl₂, a dNTP mix, primers, template DNA, a Taq polymerase and the like. In cases where the PCR product is determined following the PCR, it is convenient to add a fluorescent dye such as ethidium bromide, SYBR Green I, Pico Green or the like to the reaction solution 4. These fluorescent dyes intercalate with the DNA; accordingly, the amount of DNA produced by the PCR can be determined by using a CCD camera, micro-plate reader used for fluorescent light detection, spectrophotofluorometer or the like to detect the fluorescent light generated by the fluorescent dye. Furthermore, the PCR product may also be determined by adding a primer whose 5' terminal is labeled with a fluorescent dye or a radioactive isotope, or a dNTP mix which is labeled with a radioactive isotope (e. g., [α-³²P]dCTP).

There are no particular restrictions on the amount of the reaction solution 4, as long as this amount can be accommodated in the reaction chamber 21. In cases where the reaction solution 4 is a PCR reaction solution, the amount of the reaction solution 4 is preferably 2 to 50 µl.

For example, in a case where the diameter of the opening part 211 of the reaction chamber 21 is 4 mm, the depth of the reaction chamber 21 is 3 mm, the diameter of the bottom surface 213 of the reaction chamber 21 is 2 mm, and the distance between the first contact part 311 and the bottom surface 213 of the reaction chamber 21 and the distance between the second contact part 312 and the side surfaces 212 of the reaction chamber 21 (specifically, these distances correspond to the thickness of the reaction solution 4 in a thin configuration) are both approximately 0.1 mm, the amount of the reaction solution 4 is preferably 2 to 4 µl, and is even more preferably about 3 µl. Furthermore, in a case where the diameter of the opening part 211 of the reaction chamber 21 is 4 mm, the depth of the reaction chamber 21 is 3 mm, the diameter of the bottom surface 213 of the reaction chamber 21 is 2 mm, and the distance between the first contact part 311 and the bottom surface 213 of the reaction chamber 21 and the distance between the second contact part 312 and the side surfaces 212 of the reaction chamber (specifically, these distances correspond to the thickness of the reaction solution 4 in a thin configuration) are both approximately 0.5 mm, the amount of the reaction solution 4 is preferably 15 to 17 µl, and is even more preferably about 16 µl.

Furthermore, in a case where the diameter of the opening part 211 of the reaction chamber 21 is 5 mm, the depth of the reaction chamber 21 is 5 mm, the diameter of the bottom surface 213 of the reaction chamber 21 is 3 mm, and the distance between the first contract part 311 and the bottom surface 213 of the reaction chamber 21 and the distance between the second contact part 312 and the side surfaces 212 of the reaction chamber 21 (specifically, these distances correspond to the thickness of the reaction solution 4 in a thin configuration) are both approximately 0.1 mm, the amount of the reaction solution 4 is preferably 6 to 8 µl, and is even more preferably about 7 µl. Furthermore, in a case where the diameter of the opening part 211 of the reaction chamber 21 is 5 mm, the depth of the reaction chamber 21 is 5 mm, the diameter of the bottom surface 213 of the reaction chamber 21 is 3 mm, and the distance between the first contract part 311 and the bottom surface 213 of the reaction chamber 21 and the distance between the second contact part 312 and the side surfaces 212 of the reaction chamber 21 (specifically, these distances correspond to the thickness of the reaction solution 4 in a thin configuration) are both approximately 0.5 mm, the amount of the reaction solution 4 is preferably 34 to 36 µl, and is even more preferably about 35 µl.

### INDUSTRIAL APPLICABILITY

The present invention provides a reaction vessel which makes it possible to control the temperature of the reaction solution accommodated in the reaction chamber with a quick response, without any need for centrifuging when the reaction solution is accommodated in the reaction chamber, and which also makes it possible to cause the reaction to proceed even when the amount of reaction solution accommodated in the reaction chamber is extremely small. Furthermore, the present invention provides a reaction apparatus comprising the abovementioned reaction vessel. Moreover, the present invention provides a reaction solution temperature control method which can quickly control the temperature of the reaction solution accommodated in the reaction chamber.

## Claims

1. A reaction vessel comprising:
a reaction vessel main body which has a reaction chamber that has an opening part in the upper end and can accommodate a reaction solution;
and a cover member which can seal the opening part of said reaction chamber;
wherein said cover member has a pressing part that can press the reaction solution accommodated in said reaction chamber.

2. The reaction vessel according to claim 1, wherein said pressing part is disposed on said cover member so that the contact area between said reaction solution and said reaction chamber can be increased by the pressing of said reaction solution.

3. The reaction vessel according to claim 1 or claim 2, wherein said pressing part is disposed on said cover member so that the contact area between said reaction solution and said pressing part can be increased by the pressing of said reaction solution.

4. The reaction vessel according to any of claims 1 through 3, wherein said pressing part is disposed on said cover member so that said reaction solution can be formed into a thin configuration by the pressing of said reaction solution.

5. The reaction vessel according to any of claims 1 through 4, wherein said cover member has a first sealing part that can form a tight seal with the circumferential portion of the opening part of said reaction chamber.

6. The reaction vessel according to any of claims 1 through 5, wherein said cover member has a second sealing part that can form a tight seal with the inside surface of said reaction chamber.

7. The reaction vessel according to any of claims 1 through 6, wherein said cover member has a lifting part that makes it possible to lift said cover member.

8. The reaction vessel according to any of claims 1 through 7, wherein said reaction vessel further comprises a heat-conducting metal block which is installed so as to contact said reaction vessel main body and/or said cover member.

9. The reaction vessel according to any of claims 1 through 8, wherein said reaction solution is a PCR reaction solution, and said reaction vessel is a PCR reaction vessel.

10. A reaction apparatus comprising the reaction vessel according to any of claims 1 through 8 and a temperature control device, wherein said temperature control device is disposed so that the temperatures of said reaction chamber and said cover member can be controlled.

11. The reaction apparatus according to claim 10, wherein said reaction apparatus further comprises a cover member detachment device which can remove said cover member mounted on said reaction vessel main body from said reaction vessel main body.

12. The reaction apparatus according to claim 10 or claim 11, wherein said reaction vessel is the reaction vessel according to claim 9, and said reaction apparatus is a PCR reaction apparatus.

13. A reaction solution temperature control method comprising:
a step (a) in which a reaction solution accommodated in a reaction chamber is pressed by a pressing member; and
a step (b) in which the temperature of said reaction solution is controlled via the contact surface between said reaction solution and said reaction chamber and the contact surface between said reaction solution and said pressing member.

14. The reaction solution temperature control method according to claim 13, wherein said reaction solution is pressed in said step (a) so that the contact area between said reaction solution and said reaction chamber is increased.

15. The reaction solution temperature control method according to claim 13 or claim 14, wherein said reaction solution is pressed in said step (a) so that the contact area between said reaction solution and said pressing member is increased.

16. The reaction solution temperature control method according to any of claims 13 through 15, wherein said reaction solution is pressed in said step (a) so that said reaction solution assumes a thin configuration.

17. The reaction solution temperature control method according to any of claims 13 through 16, wherein said reaction solution is a PCR reaction solution.
